# EUROPÄISCHE PATENTANMELDUNG

(11) **EP 1 479 760 A1**
(43) Veröffentlichungstag der Anmeldung: **24.11.2004**
(21) Anmeldenummer: 03011338.5
(22) Anmeldetag: 19.05.2003
(51) Int. Cl.: C12N 5/06, C12M 3/06, C12M 3/04

(54) **Künstliches Immunorgan**

(71) Anmelder: ProBioGen AG, 13086 Berlin (DE)
(72) Erfinder: Bushnaq-Josting, Hikmat, 10439 Berlin (DE); Riedel, Marco, 13187 Berlin (DE); Marx, Uwe, 13089 Berlin (DE); Giese, Christoph, 10439 Berlin (DE)
(74) Vertreter: Helbing, Jörg, Dr. Dipl.-Chem.

(57) **Zusammenfassung**

Die Erfindung betrifft Verfahren und Vorrichtungen zur originalgetreuen Modellierung von Organfunktionen in vitro.

## Beschreibung

Die Erfindung betrifft Verfahren und Vorrichtungen zur originalgetreuen Modellierung von Organfunktionen in vitro.

### Hintergrund der Erfindung

Die Kultur von Säugerzellen ist aufgrund ihrer hohen Empfindlichkeit, relativ langsamer Vermehrung, komplexer Differenzierungsprozesse und der absoluten Sterilität als Grundbedingung grundsätzlich anspruchsvoll.

*Ex vivo* Gewebekulturen wurden in den letzten Jahrzehnten umfangreich beschrieben (Freshney). Kulturen in hoher Zelldichte oder als gewebeähnliche Strukturen erfordern eine Trennung von Versorgungsvolumen und Kulturraum, z.B. durch semipermeable Membranen. Dieses Versorgungsprinzip muss daneben effektive Lösungen für den Sauerstoffeintrag und andere Essential-Nährstoffe verwirklichen. Dies wurde über Spezialmembranen, Sauerstofftransporter in Lösung etc. gewährleistet. Darüber hinaus sollte die Kultur einer mechanischen Belastung ausgesetzt werden können, um spezifische physiologische Funktion und Strukturen (effektive Knorpelbildung) anbinden zu können.
Bei den effektivsten Bioreaktorsystemen konnte in der wissenschaftlichen Literatur die Induktion von de-novo-Selbstorganisation *in vitro* gezeigt werden. (Leber, Knorpel, Knochen, Haut usw.).
In den beschriebenen Systemen sind die Zellen in der Regel in sehr gut versorgten Zellkulturräumen immobilisiert, und für die therapeutische Applikation aus diesen entnehmbar oder im Ganzen transplantierbar (verkapselte Inselzellen). In komplexeren Systemen, wie Pioealton als Leberersatzsysteme ist auch die zeitgleiche oder zeitlich versetze Ansiedelung unterschiedlicher Zell- und Gewebetypen realisierbar.
Den jeweiligen Applikationen der beschriebenen Kultursysteme entsprechend, schafft jedoch kein Einziges die Möglichkeit, neben der de-novo-Organisation primärer Zell und Gewebestrukturen im stationären (unbewegtren) immobilisierten Zustand zeitgleich die kontinuierliche regulierbare/dimensionierbare Perfusion der immobilisierten Gewebe mit mindestens einem mobilen Gewebetyp/Zellpopulation zu gewährleisten.

Insbesondere bei Aufrechterhaltung erzeugter Mikromilieus Bedingungen/Versorgungsoptima/Stoffgradienten für immobile Zellphase und ohne Einschränkung der natürlichen/nativen Funktionen (Interaktionen) der mobilen Phase auf dem Weg durch die immobile Phase.

Die *in-vitro*-Immunisierung humaner immunkompetenter Zellen stellt letztendlich die Simulierung einer Organfunktion ex *vivo* dar und hebt damit die Anforderungen an die Zellkultur auf ein noch höheres Niveau. Es stehen verschiedene Zellkultursysteme zur Verfügung. Konventionellen zweidimensionalen Systemen, in denen die Zellen in einer Ebene meist auf dem Boden des Kulturgefäßes wachsen, stehen dreidimensionale Systeme zur Zellkultur gegenüber. Letztere bieten durch die Möglichkeit des Zellwachstums auch in die dritte Dimension eine gewebeähnliche und somit den natürlichen Bedingungen näherstehende Zellkultur.

Geeignete Zell- und Gewebekultivierungssysteme sind in der Beschreibung der folgenden Schutzrechte beschrieben:
WO 99/43788 beschreibt ein in-vitro-Modellsystem für die virale Infektion und für die Immunantwort, die aufgebaut ist aus einem auf einer flexiblen und porösen Matrix aufgebrachten Gewebeblock aus Mandeldrüse oder Lymphknoten, wobei der Gewebeblock in einem Medium kultiviert wird, dessen Oberfläche übereinstimmt mit dem Gewebeblock der Matrixoberfläche. Das Kultursystem kann zum "screenen" nach antiviralen Drogen, zur Beobachtung des Verlaufs von viralen Krankheiten und zur Beobachtung einer Immunantwort auf Antigenstimulation verwendend werden.

WO 89/11529 beschreibt einen Bioreaktor, der aus zwei Kammern besteht, einer Zufuhr- und Abfuhrkammer und einer Zellkammer, die durch eine selektivpermeable Ultrafiltrationsmembran getrennt sind. Innerhalb der Zellkammer befindet sich eine biokompatible dreidimensionale Matrix, die die tierischen Zellen einschließt. Aufgrund der Anwesenheit der biokompatiblen Matrix, weist die Zellkammer eine Gelphase auf, d. h. die biokompatible Matrix und die Zelle und eine flüssige Phase enthalten eine konzentrierte Lösung des Zellprodukts, das isoliert werden kann.

US-Patent 5,416,022 beschreibt eine kompakte, einfach zusammenbaubare Zellkultivierungsvorrichtung, die zumindest ein Zellkultivierungskompartiment aufweist, dessen Inneres den Zellkulturraum darstellt.

WO 01/04262 betrifft einen Bioreaktor sowie ein Verfahren zur Kultivierung organischen Materials, insbesondere von Zellen mittels eines Nährmediums. Die Kultivierung des organischen Materials findet dabei bei einem konstanten Nährmittelfluss statt.

DE-C-4230194 und EP-A-0590341 beschreiben einen Reaktor zur Zellkultivierung, der von zwei diskreten Hohlfasermembransystemen durchzogen ist und in dem die Kultivierung der Zellen an den von den Hohlfasern gebildeten Netzwerk erfolgt. Der Reaktor wird unter der Bezeichnung "Tecnomouse"® vertrieben.

Schließlich beschreibt EP-B-0584170 ein Kultivierungsverfahren und eine entsprechende Vorrichtung, in der Säugerzellen gleichzeitig in mehreren separaten Kulturgefäßen mit einem gemeinsamen Versorgungskreislauf kultiviert werden können, wobei der Versorgungskreislauf von den Kulturgefäßen durch eine zellrückhaltende Membran getrennt ist.

U. S. Patent 5,290,700 offenbart ebenfalls einen Reaktor, in dem wenigstens zwei verschiedene Versorungskreisläufe vorhanden sind.

Der Reaktor gemäß DE 3409501 und DE 4209501 weist zwei Kammern, eine Zellkulturkammer und eine Versorgungskammer, auf, die voneinander durch eine Membran getrennt sind. DE-A-4229334 beschreibt ein entsprechendes flaschenähnliches Kulturgefäß mit zwei Kammern.

U. S. Patent 4,242,460 offenbart eine Zellkulturvorrichtung in Spulenform, in der die Versorgungskreise durch eine semipermeable Spule und die Reaktorspule gewickelt ist. EP-A-0365313 beschreibt einen zylindrischen Bioreaktor, in dem die Versorgung durch mehrere Rohrleitungssysteme gewährleistet wird.

U. S. Patente 4,220,725 und 4,647,539 und DE 3805414 beschreiben einen Reaktor, der durch ein Bündel von semipermeablen Kapillaren durchzogen ist.

WO 93/18133 beschreibt eine Vorrichtung zur Behandlung von Zellkulturen auf platten antigenen Zellkulturträgern, die zumindest partiell gasdurchlässig ist und auf der einen Kollagenschicht mit der zu kultivierenden Zellkultur aufgebracht ist. In der DE-A-4322746 werden Hohlfasern als entsprechende Zellkulturträger vorgeschlagen.

Die vorstehend erwähnten 3D-Kultivierungsverfahren und Vorrichtungen kommen in ihrer Effizienz alle noch nicht dem natürlichen System nahe. Insbesondere ist ein kontrolliertes Anwachsen der Zellen und eine gesteuerte Versorgung des zu kultivierenden Zellmaterials mit regulatorischen Faktoren/Agenzien, insbesondere anderen Zelltypen (neben der kontinuierlichen Versorgung mit Nährstoffen) nicht gewährleistet.

### Kurzbeschreibung der Erfindung

Überraschenderweise wurde nun gefunden, dass Kultivieren von Zellen /gewebe in eine stationären Phase durch Kontakt mit einer mobilen Phase enthaltend Zellen eines unterschiedlichen Zelltyps essentiell für die Ausbildung von funktionellen Geweben sein kann, bzw. für bestimmte Gewebetypen von zentraler Bedeutung sein kann. Die Erfindung betrifft somit
(1) ein Verfahren zur Kultivierung von Zellen und/oder Gewebe, umfassend den Schritt des Kultivierens von in einem oder mehreren Kulturräumen immobilisierten Zellen unter Kontakt mit wenigstens einer zellfreien Versorgungsflüssigkeit und wenigstens einer mobilen Phase enthaltend Zellen eines anderen Zelltyps bezogen auf die immobilisierten Zellen; und
(2) eine Vorrichtung zur Kultivierung von Zellen und/oder Gewebe, umfassend wenigstens einen Kulturraum zum Ansiedeln von Zellen und/oder Gewebe, dem unterschiedliche Flüssigkeitsströme, nämlich wenigstens eine zellfreie Versorgungsflüssigkeit und wenigstens eine mobile Zellphase, kontinuierlich zuführbar sind.
Das erfindungsgemäße Verfahren (und auch die erfindungsgemäße Vorrichtung) ist unter anderem zur gezielten Gewinnung humaner Antikörper und humaner, immunkompetenter Zellen gegen ausgewählte Antigene (Zielantigene) geeignet.

### Kurzbeschreibung der Figuren

Fig. 1 zeigt eine erste Ausführungsform der erfindungsgemäßen Vorrichtung.
Fig. 2 zeigt eine zweite Ausführungsform der erfindungsgemäßen Vorrichtung.
Fig. 3 und 4 zeigen einen Ausschnitt bzw. eine Queransicht der in Fig. 2 abgebildeten Vorrichtung.

### Ausführliche Beschreibung der Erfindung

Unter "immobilisierter Zell- und Gewebekultur" im Sinne der vorliegenden Erfindung wird diejenige Kultur innerhalb der Vorrichtung verstanden, in der die Zellen durch Adhärenz an Oberflächen bzw. durch andere mechanische oder physiologische Mechanismen in einem Kompartiment (Kulturraum) der Vorrichtung gehalten werden.

Unter "mobiler Zellphase" werden all jene Zellen innerhalb der Vorrichtung verstanden, die durch einen gerichteten physikalischen, biophysikalischen oder biochemischen Prozess z. B. Flüssigkeitsströme, elektromagnetische Felder, gerichtete Drücke, Lockstoffgradienten usw. in einer bestimmten Zeit durch die Vorrichtung bewegt werden.

In dem erfindungsgemäßen Verfahren wird eine Co-Kultur von geeigneten Zellen, z.B. von primären humanen immunkompetenten Zellen, über einen Zeitraum von mehreren Monaten sichergestellt. Die Funktionalität in der Co-Kultur wird durch geeignete Zellpopulationen (zelluläre Komponenten; Zellsystem), die in einen dafür geeigneten Kulturraum und unter Verwendung einer unterstützenden Matrix eingebracht werden, gewährleistet. Ein kontinuierliches Versorgungssystem (wie z.B. das der Vorrichtung gemäß Ausführungsform (2) der Erfindung) sollte konstante Kulturbedingungen über den gesamten Kulturzeitraum gewährleisten. Die eingesetzten Zellpopulationen bilden funktionale Einheiten durch Selbstorganisation innerhalb der unterstützenden Matrix.

Die kultivierte Zellen und/oder Gewebe (nachfolgend auch kurz "Zellsystem") zeichnet sich dadurch aus, dass in ihm zellulären Strukturen und immunologische Funktionen nachempfunden werden, die den immunologisch aktiven Geweben, in Form der Keimzentren eines humanen Lymphknotens oder der humanen Milz entsprechen.
Die Ausbildung der zellulären Organisation und Mikrostruktur eines Keimzentrums wird durch ein gerichtetes Zusammenwirken verschiedener Zelltypen miteinander gewährleistet. Zu den *in vitro* nachzubildenden Funktionen zählt die gerichtete, antigeninduzierte Zellproliferation und Antikörpersekretion sowie die Ausbildung eines nachhaltig nutzbaren immunologischen Gedächtnisses.

Notwendig für die gezielte Ausbildung einer Immunantwort ist das richtige Zusammenwirken von Antigen präsentierenden, regulierend wirkenden und Antikörper produzierenden Zellen. Das Zusammenwirken wiederum setzt eine ideale Zusammenführung geeigneter Zellpopulationen voraus.

Das autologe Konzept der Zellgewinnung gemäß dem Verfahren (1) der Erfindung sichert eine bestmögliche Zell-Zellinteraktion und vermeidet ungewünschte Immunreaktionen verschiedner Zellpopulationen aufgrund unterschiedlicher Spenderherkunft. Diese müssen hinsichtlich der gewünschten Funktionen aktiv bzw. aktivierbar sein und in geeigneter räumlicher Anordnung zueinander sowie idealem Mischungsverhältnis zusammengebracht werden (z.B. DC, T-Lymphozyten, ß-Lymphozyten usw.).

In einer bevorzugten Ausführungsform der Erfindung werden das Zielantigen präsentierende Dendritische Zellen (DC, APC) zunächst in die vorbereitete, für die Kultur äquilibrierte Matrix eingebracht und ankultiviert. Mit der Matrix zusammen stellen die adhärenten DC eine quasi-stationäre Phase im Kultursystem dar. Zu dieser stationären Phase werden in geeigneter Weise T-Lymphozyten gegeben, welche in die Matrix infundiert werden, dort gerichtet migrieren, mit den DC antigenspezifisch assoziieren und damit aktiviert werden. In einem folgenden Schritt oder zeitgleich werden B-Lymphozyten infundiert, welche ihrerseits mit dem Komplex aus antigenpräsentierenden DC und entsprechend aktivierten T -Zellen assoziieren und ebenfalls antigenspezifisch aktiviert werden.
Augegehend von diesen Komplexen aus APC sowie aktivierten T- und B-Lymphozyten bildet sich zelluläre Strukturen aus, die einem Keimzentrumsbildung *in vivo* entsprechen.

Die Keimzentren *in vitro* sind durch massive Zellproliferation sowie und schließlich durch Antikörpersekretion gekennzeichnet. Dadurch nehmen sie an Zellmasse und Größe zu. Für die initiale Wechselwirkung der APC mit den mobilen, migrierenden Lymphozyten spielt die verwendete Matrix eine entscheidende Rolle: Sie stellt einerseits die Zugänglichkeit der DC für die Lymphozyten sicher, andererseits stabilisiert sie eine System von Mikrogradienten sekretorischer Botenstoffe (Cytokine) in der unmittelbaren Zellumgebung, welche für die Zell-Zellinteraktion der von zentraler Bedeutung sind. Darüber hinaus wird die umhüllende Matrix entsprechend der Ausdehnung der Keimzentren zunehmend verdrängt. Zunächst werden von den antivierten B-Lymphozyten innerhalb der Keimzentren IgM-Antikörper exprimiert und an der Zelloberfläche präsentiert . Über einen Zeitraum von mehreren Tagen vollzieht sich eine Wechsel zu Antikörpern der Subklasse IgG, die schließlich auch sekretiert werden. Parallel zum Subklassenwechsel vollzieht sich in der Keimzentrum *in vitro* durch somatische Permutation eine Affinitätsreifung des Antikörpers gegenüber dem Zielantigen. Dies geschieht durch bevorzugte Proliferation der antigenspezifischen B-Lymphozyten, deren Antikörper eine höhere Affinität zum Antigen haben und daher eine längeren Zell-Zell-Kontakt mit der APC gegenüber den anderen konkurrierenden B-Lymphozyten haben (ebenso die T- Lymphozyten untereinander). Dadurch nimmt deren Anteil an der Gesamtausschüttung antigenspezifischer Antiköper zu (klonale Expansion) und dominiert schließlich im Kulturüberstand.

Die Zahl an spezifischen B- und T-Memoryzellen (für die Induktion einer sek. Immunantwort, oder naiven B-und T-Lymphozyten (für die Induktion einer prim. Immunantwort) ist in der Co-Kultur auf die zur Verfügung stehenden APC abgestimmt. Durch ein geeignetes Verhältnis an B- und T- Lymphozyten zueinander sowie gegenüber den APC, der geeigneten Nähe der interagierenden Zellen zueinander (für erfolgreiche Migration und Assoziation) sowie der Zusammenführung einer idealen Gesamtzellmasse der Co-Kultur wird die gerichtete Ausbildung und Funktionalität der Keimzentren in Form induzierter AK-Produktion sowie Zellproliferation gewährleistet.

Als Antigenpräsentierende Zellen (APC) tragen dendritische Zellen prozessiertes Antigen an ihrer Zelloberfläche und können damit antigenspezifische T4-Helfer-Zellen (Naiv oder T-Memory) in unmittelbarer Nähe befinden aktivieren. Die Aktivierung bewirkt Cytokin-Ausschüttung (Il-2, IFN_{γ}) und klonale Expansion). Die Präsentation des Antigens erfolgt über membranständige MHC Klasse 2- Rezeptoren der DC. Für eine geeignete Präsentation wird das Antigen zuvor an Mikropartikel gebunden und den APC zur Phagocytose und Antigenprozessierung angeboten. Die Antigenzugabe erfolgt entweder vor oder während der Co-Kultivierung.
Funktionelle Dendritische Zellen werden, gemäß dem autologen Konzept, aus Blutpräparaten gewonnen und *in vitro* differenziert.

Für die Co-Kultur werden autologe T-Lymphozyten in ausreichenden Mengen und zeitgerecht bereitstellt, sobald die Differenzierung der DC bzw. die Antigenpräsentation abgeschlossen ist. Im Rahmen der Zell-Zell-Interaktion werden die regulatorischen Effekte der T -Lymphozyten genutzt. Zur Induktion einer bestehenden Immunantwort handelt es sich um T-Gedächtniszellen (Sek. Immunantwort), zur gezielten de novo-Generierung einer Immunreaktion werden dagegen naive T-Lymphozyten eingesetzt (Primäre Immunantwort). Die Bereitstellung einer ausreichende Zellmasse bei hoher Zellvitalität sowie die Verlängerung der Lebensdauer der präparierten Zellpopulation geschieht durch gezielte T -Zell-Expansion.

Ebenso werden für die Co-Kultur autologe B-Lymphozyten in ausreichender Menge, zeitgerecht bereitstellt. Auch hier können Gedächtniszellen oder naive B-Zellen eingesetzt werden, je nachdem ob eine sek. oder prim. Immunantwort induziert werden soll. Die Zell masse mit hoher Zellvitalität sowie Verlängerung der Lebensdauer der präparierten Zellpopulation wird durch B- Zell-Expansion sichergestellt.

Die überwiegend regulierend eingesetzten T-Zellen können über Zell-Zellkontakte und Cytokin-Ausschüttung geeignete spezifische B-Zellen (Naiv oder B-Memory) aktivieren und diese zu Cytokin-Ausschüttung und klonaler Expansion führen. Außerdem wird die des Zellspezifischen Immunglobulins zunächst in Form von IgM (Naiv; primäre Immunantwort) oder aber von IgG (B- Memory-Zellen, Sek. Immunantwort, bystander-Reaktion) die sich entweder oder mobil sind bzw. migrieren über Chemotaxis von den APC angezogen werden. Die zellulären Interaktionen äußern sich in Form gerichteter Zell-Migration, dem Aufbau von Zell-Zell-Kontakten, der gezielten, wechselseitig stimulierten, Ausschüttung von Cytokinen mit autound parakrin wirkenden Effekten, eine gerichtete Zellproliferation sowie die antigengetriebene Affinitätsreifung der gebildeten Antikörper (Antikörperschärfung).
Wichtige Funktionen der entstehenden Keimzentren *in vitro* bestehen daher in umfassender, gerichteter Bildung antigenspezifischer B-Zellen, der Sekretion antigenspezifischer Antikörper verschiedener Subklassen, dem gerichteter Subklassen-Wechsel und Affinitätsreifung der zielantigenspezifischen Antikörper.

Die unterstützende extrazelluläre Matrix sowie die Kulturführung stellt darüber hinaus eine entscheidend notwendige zelluläre, gewebeähnliche Selbstorganisation und Eigenkonditionierung innerhalb der Co-Kultur sicher. Sie muss die Beweglichkeit der Zellen sowie Adhäsion, Migration, Assoziation und Proliferation ermöglichen. Darüber hinaus unterstützt die Matrix, über Kulturzeiträume von mehreren Monaten hinweg, das Wachstum der Keimzentren. Dazu sind optimale Diffusionseigenschaften für Substrate und Abfallprodukte des Zellstoffwechsels gewährleistet ohne das lokale Mikromilieu an Cytokingradienten zu stören. Außerdem wird durch kontrollierte Degradation oder Verdrängung der Matrix entsprechend der Ausdehnung entstehender Keimzentren sichergestellt.

Die Langzeitkultur der entstehenden Keimzentren wird durch eine kontinuierliche Versorgung des Kulturraums (z. B. über die zellfreie Versorgungsflüssigkeit(en)) gewährleistet. Versorgungssystem, Kulturraum und Matrix sind sterilisierbar und zeigen für die Langzeitkultur eine über Monate ausreichende Prozessstabilität.

Die kontinuierliche Versorgung stellt eine ausreichende und gleichbleibende Zufuhr von Substraten sowie den Abtransport von limitierenden Stoffwechselendprodukten sicher. Dabei zirkuliert ein Mediumvolumen durch das Kultursystem und wird im Anschluss auf ideale Kulturbedingungen hinsichtlich Sauerstoffkonzentration und pH-Wert des Mediums regeneriert (äquilibriert). Mit einer gezielt einstellbaren Verdünnungsrate wird außerdem ein Anteil der zirkulierenden Kulturmediums kontinuierlich ausgetauscht. Dadurch ist eine konstante Versorgung der kultivierten Zellen mit Stoffwechsel relevanten Substraten und eine Entsorgung wachstumslimitierender Metabolite auch über lange Kulturzeiträume gewährleistet.
Außerdem lassen sich durch periodische Beprobung des zirkulierenden Kulturmediums der physiologische Zustand der Kultur beobachten (Substratverbrauch, Cytokinregression, Antikörperproduktion usw.).

Der Kulturraum ist so dimensioniert, dass sicherstellt werden kann, dass im Rahmen einer autologen Co-Kultur eine ausreichende Menge an Zellen interagieren und somit eine Immunantwort gegen beliebige Antigene induziert werden kann.
Der Kulturraum selber ist mit der wachstumsunterstützenden Matrix gefüllt und wird von mikroporösen Membranflächen durchzogen, welche eine Versorgung der Zellen mit wichtigen Substraten und Entsorgung kritischer und gewünschter Endprodukte in ausreichendem Maße gewährleistet ohne ein von der Zelle gebildetes Mikromilieu (Gradienten aus auto- und parachrinwirkenden Faktoren) zu stören.

Die ausreichende Transport von Substanzen aus dem Versorgungssystem über die Membranflächen in die Matrix und schließlich zu den stoffwechselaktiven Zellen wird gewährleistet durch:
1. Material und mikrostrukturelle Beschaffenheit der Membranflächen;
2. Geometrische Anordnung der Membranflächen zueinander;
3. Begrenzte Diffusionsstrecken innerhalb des Kulturraums; und
4. Ausreichende Transporteigenschaften der Matrix in Form guter Hydraulik und Diffusion S. Ausreichende Bioverfügbarkeit gelöster Substanzen innerhalb des Matrix-gefüllten Volumens

Das Versorgungssystem sowie der Kulturraum ermöglicht eine kontinuierliche oder auch periodische Beprobung des Kulturmediums sowie die Ernte von Zellen und zellulären Produkten, wie sekretorischer Antikörper.

Bei gleicher Zellkonzentration sollte der prozentuale Lymphozytenanteil in der Ausgangspopulation erhöht werden. Dies würde eine Annäherung an die Verhältnisse im Humanblut (M. Classen et al., *Urban* & Schwarzenberg München, Wien, Baltimore (1991)) sowie an die von Borrebaeck und Danielsson vorgeschlagene Formel bedeuten, wonach ein Verhältnis von T-Zellen : B-Zellen : akzessorischen Zellen von 2 : 1 : 0,25 als optimal eingeschätzt wird (C. A. K. Borrebaeck, 1989; L. Danielsson et al. *Immunology.* 61 (1987) 51-55). Zum Vergleich lag die Relation der genannten Zellen in der MNC-Ausgangspopulation bei 2,7:1:1 oder 0,8:0,25:0,25 und am 34. Tag bei 7:1:41 oder 0,04:0,006:0,25. Eine Separation der Zellen, insbesondere der Entzug der Monozytenpopulation wirkt sich ungünstig auf den Kulturverlauf aus.

Bezüglich der Zusammensetzung des Kulturmediums (bzw. der Versorgungflüssigkeit(en)) hat sich hinsichtlich der Mediensupplementierung ein höherer Humanserum-Zusatz als günstig zu erweisen. Auch in Anlehnung an die Ergebnisse aus den beschriebenen Untersuchungen zweidimensionaler Kulturen sollte der Zusatz von Humanserum etwa 10-12% betragen. Borrebaeck und Danielsson favorisieren den Zusatz von 10% Humanserum (C. A. K. Borrebaeck, 1989; L. Danielsson et al. *Immunology.* 61 (1987) 51-55). Im Tecnomouse®-Bioreaktor wurden dem Kulturmedium 4% HSA zugesetzt. Eine Abkehr von FKS als Medienzusatz scheint möglich zu sein. Generell sollte eine Seruminaktivierung streng eingehalten werden. Trotz der vorteilhaften dreidimensionalen Kulturführung im Bioreaktor konnte in der gegenwärtigen Entwicklungsstufe ohne T- oder B-Zellstimulantien keine effiziente in-*vitro*-Immunisierung nachgewiesen werden. Angesichts des im Verlauf unproportional hohen Lymphozytenabfalls wäre der Einsatz von IL-2, z. B. in einer Konzentration von 50 U/ml, vorteilhaft sowie zusätzlich ggf. auch der Zusatz von T-zellkonditioniertem Medium. Die eingesetzte Endkonzentration von 0,38µg HBsAg/ml lag in dem auch durch Borrebaeck und Danielsson (s.o.) als günstig eingeschätzten Konzentrationsbereich und sollte bei weiteren Optimierungsversuchen zunächst nicht verändert werden. Da hier die Zellen nicht im Organfragment vom Medium umspült, sondern im dreidimensionalen Raum über engmaschige mediumführende Hohlfasern versorgt werden, besteht kein Anlass für eine Erhöhung der o.g. IL-2-bzw. Ag-Konzentration, wie Sie durch den von Ferro und Hoffmann beschriebenen, zur Lymphozytenaktivierung erhöhten Stimulanzienbedarf impliziert wird (Ferro et al., *Immunobiology.* 188 (1993) 51-61; Hoffmann et al., *J. Immunol. Methods.* 179 (1995) 37-49).

Das erfindungsgemäße Verfahren ermöglicht folgende spezielle Verfahrensausgestaltungen:
- den Langzeitbetrieb zur Mehrfachimmunisierung, Parallelanordnungen bzw. Dimensionen für Biomassen;
- die Prozessüberwachung Beispielsweise durch Gewinnung der gebildeten spezifischen Antikörper, oder Cytokincocktails;
- die Gewinnung von humantypisch spezifische rearangierte und hypermutierte B-Lymphozyten / Plasmazellen und Gewinnung der einzelzell RNS / DNS;
- die Transfektion einer Zelllinie mit dem Antikörpergen zur Erzeugung des monoklonalen Antikörpers; und/oder
- die Gewinnung von antigenspezifischen T Suppressor / Killer Zellen für die zellsubstituierende autologe Therapie.

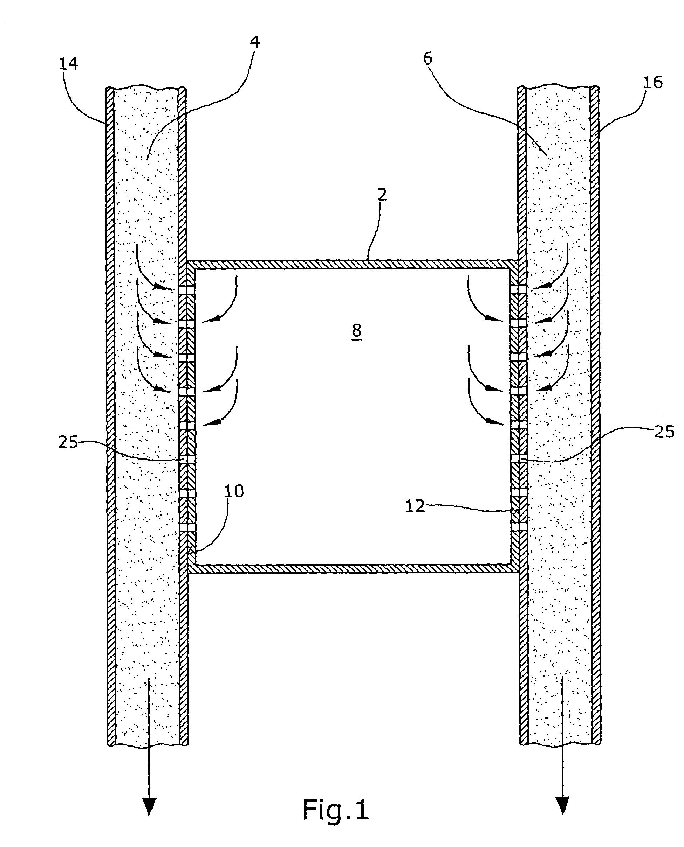

Die erfindungsgemäße Vorrichtung (2) ist insbesondere zur Durchführung des Verfahrens (1) geeignet. Besondere Ausführungsformen sind in den Figuren 1 bis 3 gezeigt.

Die Erfindung wird anhand der nachfolgenden Figuren und Beispiele näher erläutert, die jedoch den Schutzumfang der Erfindung nicht einschränken.

### Ausführliche Beschreibung der Figuren

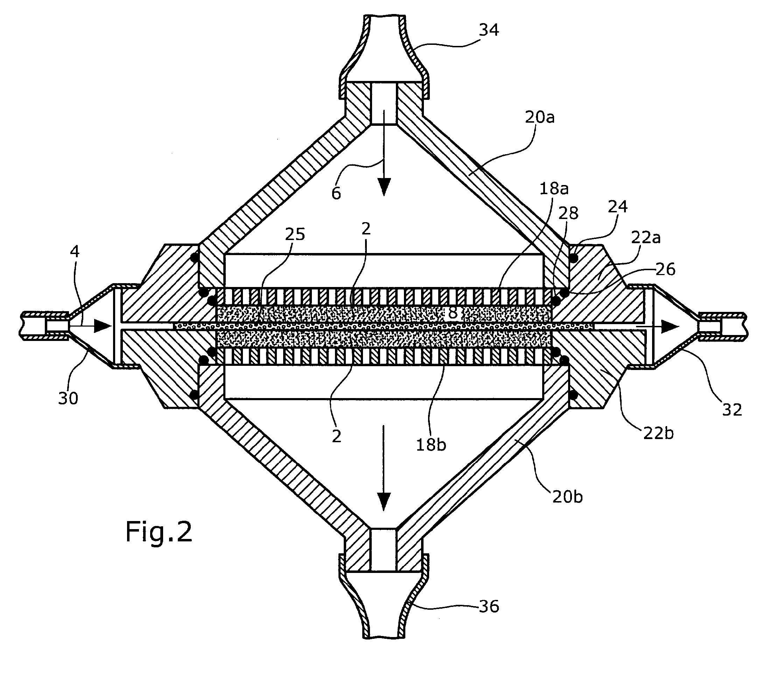

Die Figuren 1 und 2 zeigen alternative Ausführungen eine Vorrichtung zum Kultivieren von Zellen und/oder Gewebe, die einen Kulturraum 2 zum Ansiedeln von Zellen und/oder Gewebe aufweisen, dem unterschiedliche Flüssigkeitsströme 4, 6, nämlich wenigstens eine zellfreie Versorgungsflüssigkeit 4 und wenigstens eine mobile Zellphase 6 vorzugsweise kontinuierlich zuführbar sind.

In Fig. 1 sind die Flüssigkeitsströme 4, 6 parallel zu einer Umfangsfläche und entlang dieser Umfangsfläche des Kulturraums 2 in permeablen Leitungen 14, 16 vorbeigeführt.

Aufgrund der Permeabilität der Leitungen 14, 16 kann die zellfreie Versorgungsflüssigkeit 4 bzw. die wenigstens eine mobile Zellphase 6 in eine in dem Kulturraum 2 enthaltene Matrix 8 hineindiffundieren.

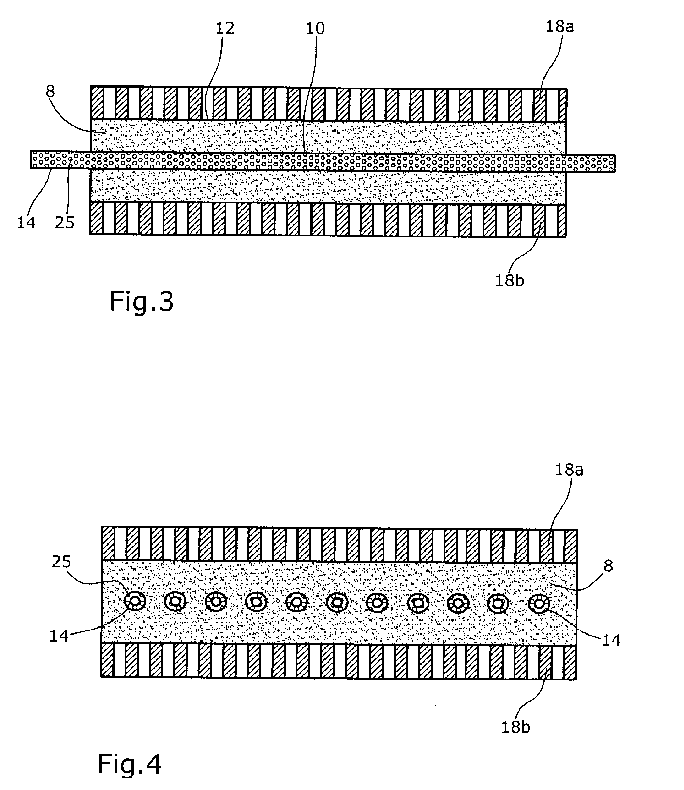

Der Stoffaustausch ist in Fig. 1 durch Pfeile angedeutet, die auf angedeutete Poren 25 der Leitungen 14, 16 zeigen. Die Leitungen bestehen vorzugsweise aus Kapillaren, nämlich Hohlfasern mit einem Durchmesser von ca. 50 - 150 µm, vorzugsweise ca. 80 - 120 µm, deren Porosität ca. 30 - 500 kDa beträgt. Die Poren 25 sind am besten in den Fig. 3 und 4 ersichtlich.

Die Leitungen 14, 16 sind an Kontaktflächen 10, 12 des Kulturraums 2 bzw. der Matrix 8 entlanggeführt, wobei in Fig. 1 repräsentativ für die Porosität der Matrix an den Kontaktflächen auch Durchgangsöffnungen des Kulturraums 2 gezeigt sind.

Verlaufen mehrere Leitungen 14 für die Versorgungsflüssigkeiten 4 nebeneinander, nämlich vorzugsweise parallel, beträgt der gegenseitige Abstand das 2- bis 10fache, vorzugsweise das 3- bis 5fache des Kapillardurchmessers.

Während in dem Ausführungsbeispiel der Fig. 1 die Leitungen 14, 16 für die mindestens eine Versorgungsflüssigkeit 4 und die mobile Zellphase 6 entlang einer äußeren Mantelfläche des Kulturraums 2 oder der Matrix 8 geführt werden, ist bei dem Ausführungsbeispiel der Fig. 2 vorgesehen, dass die Matrix 8 in einer Hohlkammer 20a, 20b vorzugsweise horizontal gehalten ist, wobei die Matrix 8 von der wenigstens einen mobilen Zellphase 6 in Querstrom durchströmt wird, während die mindestens eine Versorgungsflüssigkeit 4 durch mehrere vorzugsweise parallel im Inneren der Matrix 8 verlaufende poröse Leitungen 14, insbesondere Kapillaren, zuführbar ist.

Dabei kann die Matrix 8 durch ein Sieb 18a, 18b an der Unterseite oder beidseitig abgestützt sein. Die Porengröße des Siebes 18a, 18b beträgt vorzugsweise ca. 30 bis 100 µm.

Die Matrix 8 ist bei dem Ausführungsbeispiel der Fig. 2 ein Flächengebilde, das eine Dicke von ca. 1 bis ca. 15 mm, vorzugsweise ca. 2 bis 10 mm aufweist. Die Matrix 8 ist mit oder ohne Stützeinrichtungen 18a, 18b in der von zwei Gehäusehälften 20a, 20b gebildeten Hohlkammer gehalten, die die Matrix 8 zwischen sich aufnehmen. Die Gehäusehälften weisen Flanschteile 22a und 22b auf, die auf der einen Seite einen Anschluss 30 für die zuzuführende Versorgungsflüssigkeit 4 haben, und auf der anderen Seite einen Anschluss 32 für den Abfluss der Versorgungsflüssigkeit 4, die ggf. rezirkulierbar ist. Von dem Anschlussteil 30 verteilt sich die Versorgungsflüssigkeit 4 auf mehrere parallel angeordnete mittig durch die Matrix 8 verlaufende Leitungen 14, die am besten in Fig. 4 ersichtlich sind.

In Querrichtung zu dem Kulturraum 2 bzw. in der Matrix 8 wird in Figur 2 von oben nach unten eine mobile Zellphase 6 zugeführt und zwar eingangsseitig über einen Anschluss 34. Unterhalb der Matrix 8 kann die mobile Zellphase über einen Anschluss 36 wieder abgeführt werden und kann rezirkuliert werden. Die Flanschteile 22a, 22b sind gegen die Gehäusehälften 20a, 20b der Hohlkammer mit Hilfe von Dichtungen 24, 26, 28 abgedichtet.

### Beispiele

### Beispiel 1: Untersuchung einer dreidimensionalen Langzeitkultur humaner MNC im Tecnomouse®-Bioreaktor

A. Ergebnisse verschiedener Untersuchungen an einer dreidimensionalen Langzeitkultur humaner MNC: Humane mononukleare Zellen wurden hinsichtlich Differenzierung, Proliferation, Antikörper- und Cytokinproduktion sowie Glukoseverbrauch im zeitlichen Verlauf der Kultur charakterisiert.

Die Kultivierung erfolgte im sogenannten "Tecnomouse®-Bioreaktor", der einen miniaturisierten Perfusions-Bioreaktor darstellt, der dreidimensionale Zellkulturen mit kontinuierlicher Medienzirkulation und O₂-Versorgung ermöglicht. Es können in ihm fünf verschiedene, übereinander angeordnete Hohlfasermodule simultan betrieben werden. Die einzelnen Module werden durch das Grundgerät jeweils mit Nährmedium und einem Luft-CO₂-Gemisch versorgt. Die Nährmedienversorgung wird über einen Pumpenkopf realisiert, welcher mit fünf Segmenten eine variable Medienzufuhr zwischen 15 und 150 ml/h gestattet. Zur Begasung wird mittels einer Membranpumpe Umgebungsluft aus dem Brutschrank über einen Gasturm in das jeweilige Hohlfasermodul geführt. Über Silikonmembranen, die das entsprechende Hohlfasermodul nach oben und unten begrenzen, gelangt das Gasgemisch direkt in den Zellkulturraum. Die engmaschig parallel angeordneten Hohlfasern bilden den intrakapillären Raum. Hierüber werden Nährmedienzufuhr und Stoffwechselendprodukt-Entsorgung gewährleistet. Die eingesetzten Cuprophan-Hohlfasermembranen gestatten den Durchtritt von Molekülen bis zu einer Größe von 10 kDa. In dem die Hohlfasern umgebenden extrakapillären Raum werden die Zellen kultiviert. Zwei Ports ermöglichen die Zell- und Medieninokulation bzw. die Zellernte. Das Volumen des Extrakapillärraumes eines Hohlfasermoduls beträgt ca. 4,3 ml. Der beschriebene Reaktor stellte zum Zeitpunkt meiner Untersuchungen einen Prototyp dar, der zur Herstellung monoklonaler Antikörper als Alternativ-Methode zum Tierversuch entwickelt wurde. Die Erarbeitung einer kulturtechnisch sauberen Systembedienung sowie die Berechnung von Volumen- und Größenverhältnissen waren neben Studien zur Charakterisierung des Sauerstoffeintrages Gegenstand paralleler Untersuchungen (Maier und Nagel, Diplomarbeiten an der TU Berlin 1993; Wiesmann et al., J. Appl. Microbiol. Biotechnol. 41, 531-536 (1994)).

Kultiviert wurden zwei verschiedene Populationen aus derselben Präparation von MNC eines gesunden Human-Spenders unter folgenden Bedingungen:

| Kultur | Zeit- Raum (d) | Zellpopulation | extrakapillärer Raum (ml) | eingebrachte Zellmenge(Z) | Zellkonzentration (Z./ml) |
|---|---|---|---|---|---|
| G | 56 | MNC | ca. 4,3 | 2,4 × 10⁸ | 55,8 × 10⁶ |
| H | 34 | Lymphozyten | ca. 4,3 | 1,54 × 10⁸ | 35,8 × 10⁶ |

### Kulturmedien und Fütterungsmodus (jeweils):

### - extrakapillärer Raum:

* vor Kulturbeginn Vorinkubation mit SFM1 für 30 min.,
* Einbringen der Zellen gemeinsam mit 6 ml SFM2 und 0,5 ml HEVAC,
* einmaliger Zusatz von 2 ml SFM2 und 0,5 ml HEVAC am 34 d (nur G),

### - intrakapillärer Raum:

* kontinuierliche Perfusion mit IFM aus 5 I-Rezirkulationsflaschen, Perfusionsgeschwindigkeit = 75 ml/h,
* 14-tägiger Wechsel der Medienflaschen,
* alle 7 Tage Glutaminzugabe entsprechend der Soll-Konzentration im IFM,

### - Begasung:

* Luft-CO₂-Gemisch aus dem Brutschrank, CO₂-Anteil 5 %, Rate 1,8 l/min Untersuchungen: Kultur G Kultur H
* Messung von Glukose- und n.b. Laktatkonzentration täglich 1., 34. d
* FACS-Analyse 1., 34., d 1., 34. d
* Zellzählung 1., 34., 56. d 34. d
* ELISA (IgM, IgG, anti-Lipid A-, anti-LPS J5-, 34., 56. d 34. d anti-HBsAg-Ak) 34., 56. d
* Zytokin-Analyse (IL-1β, -1ra, -2, -4, -6, -8, TNFα)

Zur Überwachung der Stoffwechselsituation, des Nährstoffangebotes sowie zur frühzeitigen Erkennung möglicher Infektionen wurden im ZKÜ der MNC-Population täglich die Glukose- und Laktatkonzentration gemessen. Zellzählungen wurden mit der Ethidiumbromid-Acridinorange-Methode durchgeführt. Eine vergleichende FACS-Analyse beider Populationen am 1. und 34.Tag der Kultur sollte während der Kultur aufgetretene Differenzierungs- und Proliferationsvorgänge aufdecken. Es wurden die folgenden Antikörper eingesetzt:

| Untersuchungsschritt | Antikörper-Gemisch | | |
|---|---|---|---|
| 01 | Autofluoreszenz , | | |
| 02 | CD 45-F , | CD 14-PE , | |
| 03 | CD 2-F, | CD 5-PE , | CD 4- und CD 8-PerCP , |
| 04 | BMA 031-F , | CD 5-PE , | CD 4- und CD 8-PerCP , |
| 05 | CD 11a-F , | CD 5-PE , | CD 4- und CD 8-PerCP , |
| 06 | CD 25-F , | CD 5-PE , | CD 4- und CD 8-PerCP , |
| 07 | CD 45 RA-F , | CD 5-PE , | CD 4- und CD 8-PerCP , |
| 08 | CD 45 R0-F , | CD 5-PE , | CD 4- und CD 8-PerCP , |
| 09 | CD 71-F , | CD 5-PE , | CD 4- und CD 8-PerCP , |
| 10 | HLA-DR-F , | CD 5-PE , | CD 4- und CD 8-PerCP , |
| 11 | LECAM 1-F , | CD 5-PE , | CD 4- und CD 8-PerCP |

Bei der FACS-Analyse am 34. Tag kamen zusätzlich CD 3- und CD 20-PerCP-Ak zum Einsatz. Es wurden die folgenden Ergebnisse erhalten:

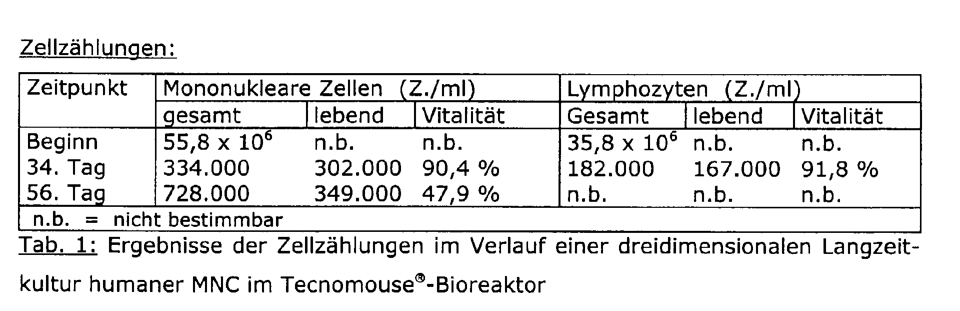

**Tab. 2:**

| Anteil von Lymphozyten und Makrophagen bzw. dendritischen Zellen an der jeweiligen Gesamtpopulation *FACS-Analysen:* | | | |
|---|---|---|---|
| Anteil an der Gesamtpopulation: | Mononukleare Zellen, Kulturbeginn | Mononukleare Zellen, 34. Tag | Lymphozyten, 34. Tag |
| Lymphozyten: Makrophagen/dendritische Zellen: | 81 % 19 % | 17 % 83 % | 8 % 92 % |
| vitale Makrophagen/dendritische Zellen: | 12 % | 20 % | 42 % |
| Anmerkung: Die hier genannte Aufteilung in Subpopulationen erfolgte über die unterschiedliche Zellablenkung im Vorwärts- und Seitwärts-Scatter der FACS-Gerätes aufgrund unterschiedlicher physikalischer Zelleigenschaften sowie über die unterschiedliche Fluoreszenz der Zellen nach Markierung mit dem Ak-Gemisch CD 45-F/CD 14-PE. | | | |

Eine Zuordnung der untersuchten Antigene zu den entsprechenden Zelltypen bzw. Aktivierungszuständen ist in der Tabelle 1-1 im Abschnitt 1.3.1.2. aufgeführt.

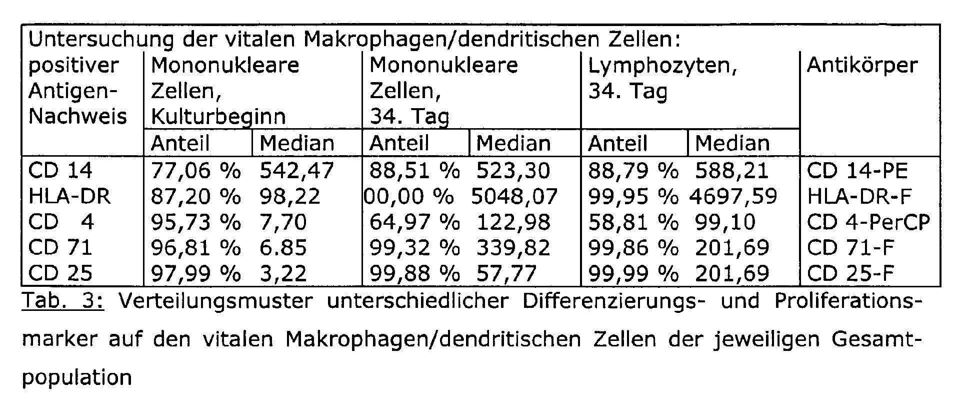

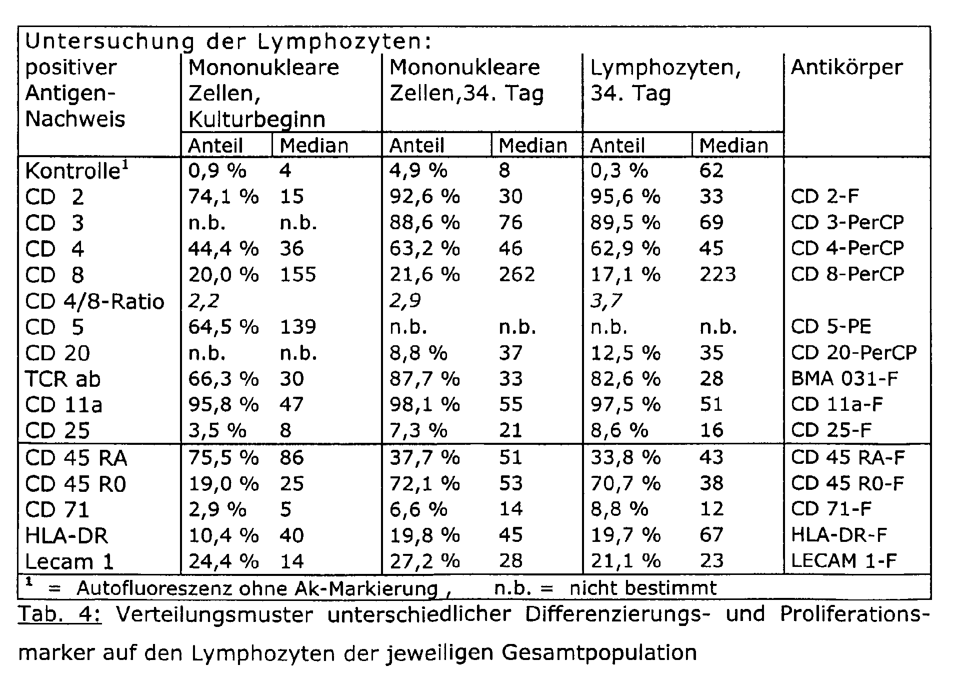

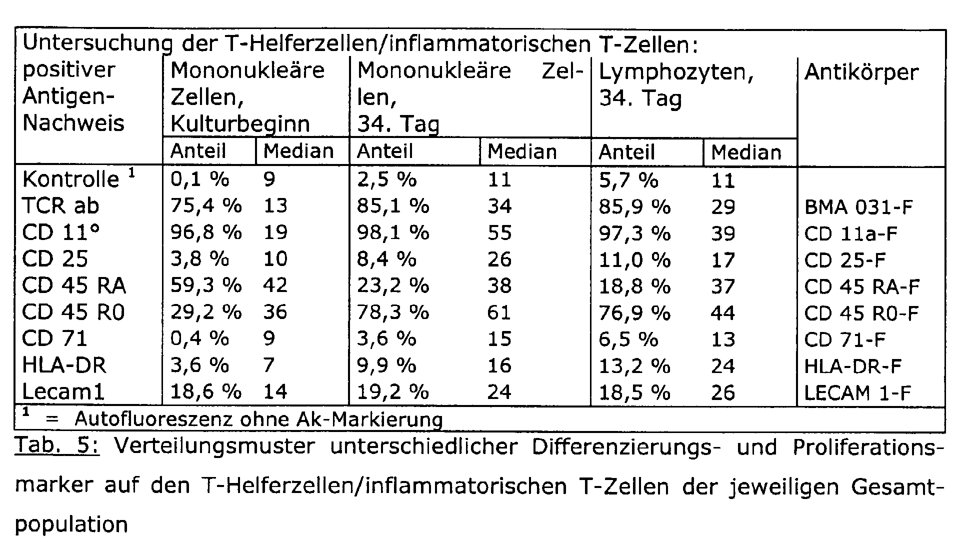

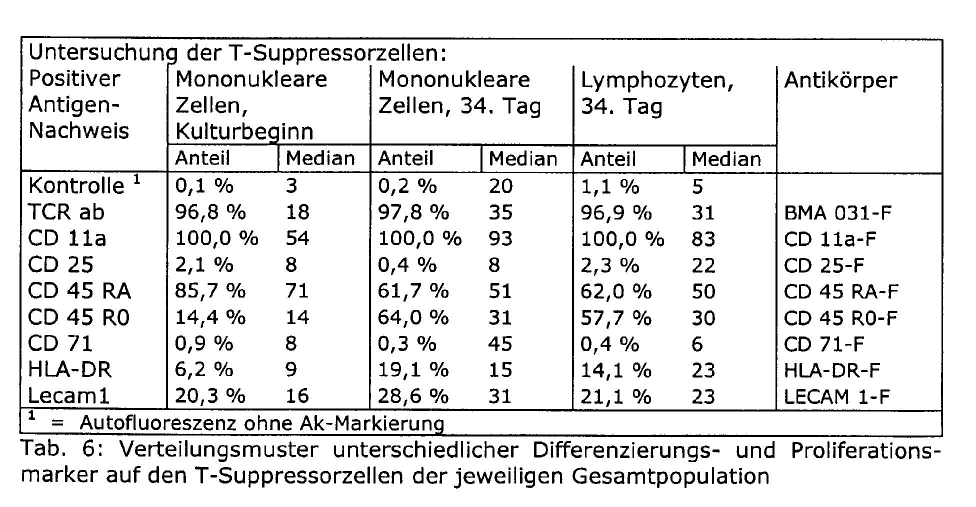

**Tab. 7:**

| Nachweis verschiedener Antikörper im ZKÜ der genannten Populationen sowie im Serum des MNC-Spenders | | | | |
|---|---|---|---|---|
| ELISA: | | | | |
| Antikörper-Nachweis | Serum des MNC-Spenders | ZKÜ der MNC, 34. Tag | ZKÜ der MNC, 56. Tag | ZKÜ der Lymphozyten, 34. Tag |
| IgM | ++ | + | + | + |
| IgG | +++ | ++ | ++ | ++ |
| Lipid-A-Ak | + | -- | -- | -- |
| LPS-J5-Ak | + | -- | -- | -- |
| anti-HBs-Ak | +++ | ++ | + | + |
| Legende: "--" = 0 - 0,1; "+" = 0,1 - 1; "++" = 1 - 10; "+++" = 10 - 100 µg/ml | | | | |

**Tab. 8:**

| Nachweis verschiedener Cytokine im ZKÜ der genannten Populationen | | | |
|---|---|---|---|
| Cytokin-Nachweis: | | | |
| Cytokin-Nachweis (pg/ml) | Mononukleare Zellen, 34. Tag | Mononukleare Zellen, 56. Tag | Lymphozyten, 34. Tag |
| IL-8 | 3879 | 492 | 4931 |
| IL-1ra | 38020 | 14400 | 36800 |
| IL-1β, IL-2, IL-4, IL-6, TNFα | 0 | 0 | 0 |

Glukose- und Laktatkonzentration: Bei den täglichen Messungen der Glukose- und Laktatkonzentration über die gesamte Dauer des Kulturverlaufes lag der Glukosegehalt mindestens bei 12,3 mmol/l. Die maximale Laktatkonzentration betrug 1,9 mmol/l.

B. Diskussion der Ergebnisse einer dreidimensionalen Langzeitkultur humaner MNC: Die Ergebnisse zeigen, dass im beschriebenen Tecnomouse® -Bioreaktor eine 8-wöchige Kultur von humanen immunkompetenten Zellen möglich ist. Sowohl in der MNC- als auch in der Lymphozytenpopulation war nach 5-wöchiger Kultur eine Vitalität von ca. 90% nachweisbar.

Bezüglich der prozentualen Verteilung immunkompetenter Subpopulationen entsprach die Zusammensetzung der MNC-Ausgangspopulation in etwa der bei Classen beschriebenen Normalverteilung im peripheren Humanblut (M. Classen et al., *Urban & Schwarzenberg* München, Wien, Baltimore (1991)). In den ersten 5 Wochen der Kultur veränderte sich die Zellverteilung deutlich zugunsten der Makrophagen und dendritischen Zellen. Die detaillierte Auswertung der FACS-Analysen lässt erkennen, dass in beiden Kulturvarianten Proliferationen bzw. Aktivierungsprozesse stattgefunden haben. Die Proliferationsmarker HLA-DR, CD71 und CD 25 stiegen in allen Subpopulationen über die ersten 5 Wochen an. Die einzige Ausnahme bildeten hierbei die T-Suppressorzellen, bei denen lediglich ein HLA-DR-Anstieg sichtbar wurde. Trotz des deutlichen Abfalls der Lymphozytenzahlen wird innerhalb der Lymphozytenpopulation eine Proliferation bzw. Aktivierung sichtbar. In beiden Kulturvarianten ließ sich ein Anstieg der T-Zell-Marker CD2, CD4 und TCRαβ nachweisen. Ein CD8-Anstieg wird erst unter Berücksichtigung der Medianwerte erkennbar, so dass zusammenfassend ein Entwicklungsschub aller T-Zell-Subpopulationen abgeleitet werden kann. Diese Ableitung wird durch die Reduktion naiver T-Zellen (CD45RA) bei gleichzeitigem Anstieg von T-Memoryzellen bzw. aktivierten T-Zellen (CD45R0) unterstützt. Der kontinuierliche Nachweis von CD11a und Lecam1 spricht für die funktionierende Expression von Adhäsionsmolekülen.

Die im Verlauf mittels ELISA untersuchten Ak-Konzentrationen haben gezeigt, dass selbst bis zur 8. Kulturwoche hohe Konzentrationen an Antikörper nachzuweisen sind (s. Tab. 7).

Die Cytokine IL-8 und IL-1ra waren über die gesamte Kulturdauer nachweisbar. Die Auswertung der täglichen Bestimmung von Glukose und Laktat im rezirkulierenden Medium (intrakapillärer Raum) ergab einen über den gesamten Kulturverlauf stabilen Glukose- bzw. Laktatspiegel in den angegebenen Konzentrationen, was auf ein stabiles Nährstoffangebot ohne kritische Akkumulation von Stoffwechselendprodukten schließen lässt.

Der Abfall der CD45 RA/RO-Ratio sowie die Abnahme des IgM/IgG-Quotienten im zeitlichen Verlauf lassen erkennen, dass bereits in recht einfachen Untersuchungsansätzen eine primäre und auch sekundäre Immunisierung möglich zu sein scheint.

### Beispiel 2: Nachweis von Migration von Zellen in einem perfundierten Kultursystem

Das Beispiel beschreibt die Entwicklung eines Modells eines kapillären Blutgefäßes zur in vitro Untersuchung von Zellmigrationen. Es wird gezeigt, dass isolierte neutrophile Granulozyten aus einem simulierten Blutstrom im Inneren der Kapillare zu einem durch Entzündungsmediatoren imitierten Entzündungsherd migrieren (Chemotaxis).
A. Isolation und Kultivierung der Endothelzellen: Für alle Versuche wurden humane Endothelzellen aus der Nabelschnurvene (human umbilical vein endothelial cells - HUVEC) verwendet. Die Nabelschnüre stammten aus der Frauenklinik des Universitätsklinikums Leipzig und aus der Entbindungsstation des Kreiskrankrenhauses Wurzen. Sie wurden sofort nach der Geburt unter sterilen Bedingungen an den Enden abgeklemmt und bis zur Abholung bei 4 °C aufbewahrt. Die Isolation der Endothelzellen erfolgte innerhalb von 24 h nach der Abnabelung.
   Vor Isolation der Endothelzellen wurden die Nabelschnüre zur äußerlichen Desinfektion 30 s in 70%igen Ethanol getaucht. Anschließend wurden die Enden vor den Nabelschurklemmen mit einem Skalpell durchtrennt. Die Nabelschurvene wurde mit phosphatgepufferter Saline gespült, bis der Abfluss klar war. Anschließend wurde die Nabelschnurvene mit 0,02%iger Kollagenaselösung (aus *Clostridium histolyticum,* SIGMA) gefüllt und 45 Minuten bei 37 °C inkubiert. Die Zellsuspension wurde aus der Vene entnommen und mit 50 ml phosphatgepufferter Saline nachgespült. Nach Zentrifugation (15 min, 1000 min⁻¹) wurde die Zellen in Kulturmedium überführt.
   Sofern nicht anders vermerkt wurden die Zellen in DMEM (SIGMA) mit Zusatz von 10 % hitzeinaktiviertem fötalem Kälberserum (SIGMA), 44mM NaHCO₃, 25 mM HEPES, 10 U/ml Heparin (seromed), 10 ng/ml basic fibroblast growth factor (bFGF, SIGMA) und 100 µg/ml endothelial cell growth factor (ECGF, Boehringer) kultiviert. Die Kultivierung erfolgte in 75 cm²-Kulturflaschen (nunc), die durch dreistündiges Inkubieren mit einer 2%igen Gelatinelösung (SIGMA) vorbeschichtet wurden. Es wurden 10 ml Medium oben genannter Zusammensetzung je Kulturflasche verwendet. Der Mediumwechsel erfolgte alle 3 - 4 Tage. Nach Erreichen von 80-100%iger Konfluenz wurden die Zellen trypsiniert (Trypsin-EDTA, SIGMA) und im Verhältnis 1:2 gesplittet. Für Versuche wurden Zellen der 2.-4. Passage verwendet.
B. Testung der extrazellulären Matrix: Zur Auswahl einer geeigneten extrazellulären Matrix wurden kommerziell erhältliche Zellkultureinsätze mit unporösen Polycarbonatmembranen (nunc) verwendet. Sie wurden mit folgenden Materialien beschichtet: Gelatine, 6, 8 und 10 µg/cm² Kollagen I. Parallel wurden unbeschichtete Membranen eingesetzt. Zur Gelatinebeschichtung wurden 200 µl Gelatinelösung (SIGMA) gegeben, nach 3 Stunden die Flüssigkeit aspiriert und die Einsätze über Nacht im Brutschrank getrocknet. Zur Kollagenbeschichtung wurden 100, 133 und 166 µl einer 0,002 % (w/v) Kollagenlösung (SIGMA) auf die Membran gegeben und für 12 h im Brutschrank inkubiert. Nach dem Absaugen der Flüssigkeit wurden die Einsätze zur Neutralisation der Säure mit je 4 ml PBS gewaschen. Je 200 µl Endothelzellsuspension wurden in die Kultureinsätze gegeben, so daß die Zelldichte 1 x 104 Zellen betrug. Die umgebenden Näpfe wurden mit 500 µl Kulturmedium gefüllt. Alle 3 Tage wurden innerhalb des Kultureinsatzes 200 µl Medium ausgetauscht. Ab dem dritten Tag wurden täglich 2 Ansätze abgebrochen, trypsiniert und Zellzahl und Vitalität bestimmt.
C. Ermittlung der Verdopplungszeit und maximalen Zelldichte: In gelatine-beschichteten 24-well-Platten (nunc) wurden Endothelzellen der verschiedener Passagen in einer Dichte von 5 x 10³, 1 × 10⁴ und 2 x 10⁴ Zellen/cm² eingesät. Ab dem dritten Tag erfolgte täglicher Mediumwechsel. Pro Tag wurden jeweils 2 Ansätze abgebrochen, die Zellen trypsiniert und unter dem Lichtmikroskop ausgezählt. Die Detektion lebender Zellen erfolgt durch Ausschluß des Farbstoffes Trypanblau.
D. Bau des Kultursystems: Zur Vorbereitung wurden 2 ml Pipetten auf 35 mm Länge zugeschnitten und die Löcher für die extrakapillären Ports eingebracht. Die Verklebung sämtlicher Komponenten erfolgte mit handelsüblichem Zweikomponenten-Kleber. Für die Ports wurden Luerstecker (female tee) verwendet.
   Die Mikrokapillare wurde so in einen intrakapillären Port eingeklebt, daß sie im Hohlzylinder zentriert ist. Anschließend wurde die Kapillare durch den Hohlzylinder und den zweiten intrakapillären Port eingefädelt. Anschließend wurden beide intrakapilläre Ports mit dem Hohlzylinder verklebt. Die Kapillare wurde vorsichtig gespannt, und in den zweiten intrakapillären Port eingeklebt. Zum Abschluss wurden sämtliche extrakapilläre Ports in den Hohlzylinder eingeklebt. Eine schematische Zeichnung und ein Modell des Bioreaktors ist in Fig. 2 gezeigt.
E. Besiedelung des Kultursystems: Die Reaktoren wurden durch die Zentralsterilisation der Universitätsklinik Leipzig mittels Gassterilisation sterilisiert. Anschließend wurden die Reaktoren mit 200 ml sterilem Aqua dest. über die intrakapillären Ports gespült. Zur Beschichtung der Kapillare mit einer extrakapillären Matrix wurde der Reaktor über die extrakapillären Ports mit Kollagen-I-Lösung befüllt und 24 h bei einmaligem Wechsel der Kollagenlösung inkubiert. Die Säure wurde durch Spülen mit 50 ml sterilem PBS neutralisiert. Anschließend wurde der Hohlzylinder mit komplettem Kulturmedium befüllt.
   Für die Versuche wurden Zellen der zweiten und dritten Passage verwendet. Die Monolayer der Vorkultur wurden bei ca. 80 % Konfluenz trypsiniert und auf eine Zelldichte von, sofern nicht anders vermerkt, 1 x 10⁷ Zellen/ml eingestellt. Die Zellsuspension wurde über die intrakapillären Ports in das Innere der Kapillare eingebracht. Zur gleichmäßigen Verteilung der Zellsuspension wurden die Kultursysteme im Inneren einer speziell hergerichteten Rollerflasche eingespannt und 4 h bei einer Geschwindigkeit von 1 min⁻¹ gerollt. Zur Ausbildung des Monolayers wurden die Kultursysteme anschließend 7 Tage im Brutschrank inkubiert.
   Die Kultursysteme wurden nach oben beschriebener Vorgehensweise mit Endothelzellen besiedelt. Dabei wurde allerdings die Animpfsuspension auf eine Zelldichte von 2 x 10⁷ Zellen eingestellt. Nach der Besiedelung wurde das Kulturmedium im extrakapillärem Raum durch 2 % Methylcellulose ersetzt und die Kultursystem 90 min im Brutschrank inkubiert. Anschließend wurden das Innere der Kapillare mit Kulturmedium durchströmt, wobei eine Fließgeschwindigkeit von 0,3 und 1 ml/h eingestellt wurde. Nach Durchströmen der Kapillare wurde das Medium aufgefangen, und jeweils im Durchsatz jeder Stunde die Zahl lebender und toter Endothelzellen bestimmt.
E. Isolation neutrophiler Granulozyten und Lymphozyten aus buffy coat: Die Isolation der Leukozyten erfolgte aus buffy coat, die durch die Blutspende des Instituts für Klinische Immunologie und Transfusionsmedizin der Universität Leipzig zur Verfügung gestellt wurden. Zur Granulozytenisolierung wurden die buffy coat 1:10 mit PBS verdünnt und jeweils 30 ml der Mischung mit 10 ml Dextran-Lösung gemischt. Nach 1 h wurde der erythrozytenarme Überstand abgenommen. Jeweils 24 ml des Überstandes wurden auf 18 ml Ficoll geschichtet und 40 min bei 1600 min⁻¹ ohne Bremse zentrifugiert. Alle Zonen bis zum Pellet wurden verworfen, das 2 mal mit 50 ml PBS gewaschen. Nach Bestimmung der Zellzahl wurde die Zelldichte auf 5 x 10⁶ Zellen/ml eingestellt.
   Zur Lymphozytenisolation wurde der buffy coat 1:10 mit PBS verdünnt und je 24 ml auf 18 ml Ficoll geschichtet. Nach 40 min Zentrifugation bei 1600 min⁻¹ wurde der Zellring zwischen Serum- und Ficollphase abpipettiert und 2 mal mit PBS gewaschen. Die Zellzahl wurde bestimmt und eine Zelldichte von 2 x 10⁶ Zellen eingestellt.
   Die eingestellten Zellzahlen entsprechen der mittleren Dichte dieser Zellpopulationen im menschlichen Blut. Die Identität der Zellen wurde mittels Pappenheim-Färbung von Zellausstrichen überprüft.
G. Einsatz des Kultursystems als Entzündungsmodell: Nach Besiedelung des Kultursystems wurde das Kulturmedium im extarkapillären Raum durch 2%ige Methylzellulose ersetzt. Zur Simulation eines lokalen Entzündungsherdes wurden am mittleren extrakapillären Portes 10 µl einer Lösung von IL1β (c=10 U/ml) platziert. Zur Ausbildung eines Cytokingradienten in der Methylzellulose wurden die Reaktoren anschließend 90 min im Brutschrank inkubiert. Als Negativkontrolle dienten Reaktoren ohne Cytokinstimulation, sowie unbesiedlete Reaktoren mit und ohne Cytokinstimulation.

Mittels Infusionspumpen wurden Suspensionen von Granulozyten und Lymphozyten über die intrakapillären Ports durch das innere der Kapillare perfundiert. Nach 90 min Perfusion wurde die Methylzellulose aus dem extrakapillären Raum entfernt und nach Zellen durchmustert.

Die Kapillaren wurden trypsiniert und Anzahl und Vitalität der Endothelzellen bestimmt. In drei Parallelexponaten konnte eine massive Infiltration migrierender Granulocyten in der Größenordnung von 30 000 nachgewiesen werden.

## Patentansprüche

1. Verfahren zur Kultivierung von Zellen und/oder Gewebe, umfassend den Schritt des Kultivierens von in einem oder mehreren Kulturräumen immobilisierten Zellen unter Kontakt mit wenigstens einer zellfreien Versorgungsflüssigkeit und wenigstens einer mobilen Phase enthaltend Zellen eines anderen Zelltyps bezogen auf die immobilisierten Zellen.

2. Verfahren nach Anspruch 1, wobei
(i) die Kulturräume eine Matrix aufweisen, die vorzugsweise ausgewählt ist aus Gelen, poröse Materialien wie offenporigen Schäumen usw., Geweben, Vliesen usw.; und/oder
(ii) die immobilisierten Zellen Säugerzellen, insbesondere humane Zellen sind, wobei hämatopoetische Stammzellen, dendritische Zellen, Monocyten, Makrophagen oder andere primäre oder transformierte Zellen besonders bevorzugt sind; und/oder
(iii) die zellfreie Versorgungsflüssigkeit eine für die Zellen und/oder das Gewebe angepasste Nährlösung ist; und/oder
(iv) die Zellen der mobilen Phase, Zellen mit regulatorischen oder später Antikörperbildenden Eigenschaften sind und insbesondere mononukleare Zellen, wie T- und B- Lymphozyten usw. sind; und/oder
(v) die zellfreie Versorgungsflüssigkeit und die mobile Phase kontinuierlich austauschend sind; und/oder
(vi) die zellfreie Versorgungsflüssigkeit und weiterhin zum Abtransport der von dem Gewebe und/oder den Zellen gebildeten Metaboliten/Zellprodukte dient; und/oder
(vii) die zellfreie Versorgungsflüssigkeit und die mobile Phase eine unterschiedliche Strömungsgeschwindigkeit aufweisen.

3. Verfahren nach Anspruch 1 oder 2, wobei die Matrix des Kulturraums eine Porosität aufweist, die eine ausreichende Durchströmung mit der mobilen Phase, die Migration der Zellen in der Matrix und die Ausbildung von lokalen Stoffgradienten (Mikromilien) ermöglicht und vorzugsweise eine Porosität von über 30 µm aufweist.

4. Verfahren nach einem oder mehreren der Ansprüche 1 bis 3, wobei die in dem Kulturraum kultivierten Zellen aus einer bzw. mehreren Zellpopulationen bestehen, die zur Selbstorganisation und Ausbildung von Stoffgradienten befähigt sind.

5. Verfahren nach Anspruch 1, wobei (i) die mobile Phase den Kulturraum in einer Art und weise durchströmt, dass eine organtypische Interaktion aller Zell- und Gewebepopulationen im Kultursystem gewährleistet ist (B-Lymphozyten mit T-Lymphozyten und DC) und/oder Subpopulationen der mobilen Phase vorzugsweise Antigenspezifische Lymphozyten, zeitweilig zur Interaktion immobilisiert werden, vorzugsweise unter Ausbildung von Antigeninduzierten Keimzentren; und/oder (ii) dass eine Migration von Zellen quer oder entgegen der Stromrichtung gewährleistet ist.

6. Verfahren nach einem oder mehreren der Ansprüche 1 bis 5, das zur gezielten Gewinnung humaner Antikörper und humaner, immunkompetenter Zellen gegen ausgewählte Antigene (Zielantigene) geeignet ist.

7. Vorrichtung zum Kultivieren von Zellen und/oder Gewebe, umfassend wenigstens einen Kulturraum (2) zum Ansiedeln von Zellen und/oder Gewebe, dem unterschiedliche Flüssigkeitsströme, nämlich wenigstens eine zellfreie Versorgungsflüssigkeit (4) und wenigstens eine mobile Zellphase (6), kontinuierlich zuführbar sind.

8. Vorrichtung nach Anspruch 7, **dadurch gekennzeichnet, dass**
(i) der Kulturraum (2) eine Matrix (8) enthält; und/oder
(ii) die Flüssigkeitsströme (4, 6) an für Zellen permeablen Kontaktflächen (10, 12) mit dem Kulturraum (2) oder der Matrix (8) entlang der Umfangsflächen des Kulturraums (2) oder der Matrix (8) strömen oder durch die Matrix (8) hindurch diffundieren oder strömen; und/oder
(iii) die Flüssigkeitsströme (4, 6) dem Kulturraum (2) oder der Matrix (8) über permeable Leitungen (14, 16) zuführbar sind, wobei wenigstens eine der Leitungen für die mobile Zellphase permeabel ist; und/oder
(iv) die wenigstens eine Versorgungsflüssigkeit (4) über permeable Leitungen (14, 16), insbesondere Kapillaren usw., durch oder entlang des Kulturraums (2) oder der Matrix (8) zuführbar sind.

9. Vorrichtung nach Anspruch 8, **dadurch gekennzeichnet, dass** die Hohlräume der Matrix (8) einen Durchmesser von mindestens dem Doppelten des Zelldurchmessers der Zellen der mobilen Phase aufweisen;

10. Vorrichtung nach Anspruch 8 oder 9, **dadurch gekennzeichnet, dass** die Matrix (8) ein Flächengebilde mit vorzugsweise einer Dicke von ca. 1 bis 15 mm, besonders bevorzugt ca. 2 bis 10 mm, ist.

11. Vorrichtung nach Ansprüchen 8 bis 10, **dadurch gekennzeichnet, dass**
(i) die wenigstens eine mobile Zellphase (6) die Matrix (8) im Querstrom durchströmt; und/oder
(ii) die vorzugsweise horizontale Matrix (8) in einer Hohlkammer (20a, 20b) gehalten ist, durch die die wenigstens eine mobile Zellphase (6) im Querstrom strömt;. und/oder
(iii) die Matrix (8) einseitig oder beidseitig von einer für Zellen durchlässigen Stützeinrichtung (18a, 18b), insbesondere ein Sieb abgestützt ist, wobei die Porengrösse der Stützeinrichtung (18a, 18b) vorzugsweise ca. 30 bis 100 µm beträgt.

12. Vorrichtung nach den Ansprüchen 8 bis 11, **dadurch gekennzeichnet, dass** die mindestens eine Versorgungsflüssigkeit (4) der Matrix (8) durch mehrere vorzugsweise parallel im Inneren der Matrix (8) verlaufende poröse Leitungen (14), insbesondere Kapillaren, zuführbar ist, wobei vorzugsweise der Durchmesser der Leitungen (Kapillaren) ca. 50 bis 150 µm, besonders bevorzugt ca. 80 bis 120 µm beträgt, die Porosität der Leitungen (14) (Kapillaren) ca. 30 bis 500 kDa beträgt und/oder der gegenseitige Abstand der vorzugsweise parallel verlaufenden Leitungen (14) (Kapillaren) den 2 bis 10-fachen, vorzugsweise den 3 bis 5-fachen Kapillardurchmesser beträgt.

13. Vorrichtung nach einem der Ansprüche 8 bis 10, **dadurch gekennzeichnet, dass** die Leitungen (14, 16) für die mindestens eine Versorgungsflüssigkeit (4) und die der mobilen Zellphasen entlang einer äußeren Mantelfläche des Kulturraums (2) oder der Matrix (8) geführt werden.

14. Vorrichtung nach einem oder mehreren der Ansprüche 7 bis 11, **dadurch gekennzeichnet, dass**
(i) in der Matrix (8) ein für eine gewebeähnliche Kultur geeignetes Mikromilieu gebildet ist, dass die Ausbildung von Stoffgradienten sowie die optimale Versorgung dieser Phase, nämlich über eine kontinuierliche bzw. diskontinuierliche gerichtete regulierbare Durchströmung des in der Matrix (8) befindlichen immobilen Zellphase mit mindestens einer Zell- und Gewebepopulation, gewährleistet; und/oder
(ii) die mobile Zellphase rezirkulierbar oder ohne Sedimentation im restlichen Kreislauf direkt durchführbar ist; und/oder
(iii) der Kulturraum (2) bei Bedarf mit Antigen beaufschlagbar ist; und/oder
(iv) eine Einrichtung vorgesehen ist, mit der eine Zell- und oder Produkternte vorzugsweise mittels mechanischer dynamischer und/oder enzymatischer Mechanismen durchführbar ist.
